# EUROPEAN PATENT SPECIFICATION

(11) **EP 2 296 680 B1**
(45) Date of publication and mention of the grant of the patent: **20.02.2013**
(21) Application number: 08873375.3
(22) Date of filing: 11.11.2008
(51) Int. Cl.: A61K 36/185, A61P 9/00

(54) **LABISIA PUMILA EXTRACTS FOR REDUCING THE RISK OF CARDIOVASCULAR DISEASES**
LABISIA PUMILA-EXTRAKTE ZUR VERRINGERUNG DER GEFAHR VON HERZ-KREISLAUF-ERKRANKUNGEN
EXTRAITS DE LABISIA PUMILA POUR RÉDUIRE LE RISQUE DE MALADIES CARDIOVASCULAIRES

(30) Priority: 19.03.2008 MY 0800737
(43) Date of publication of application: 23.03.2011
(73) Proprietor: Government of Malaysia, as represented by The Ministry of Science, Technology and Innovation, Malaysia, 62662 Putrajaya (MY)
(72) Inventor: WAN MOHAMUD, Wan Nazaimoon, 50588 Kuala Lumpur (MY)
(74) Representative: Lord, Hilton David
(86) International application number: PCT/MY2008/000129
(87) International publication number: WO 2009/116848

(56) References cited:
- KR-A- 20090 113 723
- US-A1- 2007 082 069
- FAZLIANA M ET AL: "Labisia pumila extract regulates body weight and adipokines in ovariectomized rats", MATURITAS, ELSEVIER SCIENCE PUBLISHERS IRELAND LTD, IR, vol. 62, no. 1, 20 January 2009 (2009-01-20), pages 91-97, XP025898229, ISSN: 0378-5122, DOI: DOI:10.1016/J.MATURITAS.2008.10.004 [retrieved on 2008-12-02]
- AL-WAHAIBI, A. ET AL.: 'Effect of Water Extract of Lubisia pumila Var Alata on Aorta of Ovariectomized Sprague Dawley Rats' JOURNAL OF NUTRITION vol. 7, no. 2, 2008, pages 208 - 213
- SHAHRIM, Z. ET AL.: 'The In Vivo Rodent Micronucleus Assay of Kacip Fatimah (Lubisia pumila) Extract' TOPICAL BIOMEDICINE vol. 23, no. 2, 2006, pages 214 - 219
- GRUNDY, S.M. ET AL.: 'Obesity, Metabolic Syndrome, and Cardiovascular Disease' THE JOURNAL OF CLINICAL ENDOCRINOLOGY AND METABOLISM vol. 89, no. 6, 2004, pages 2595 - 2600
- WILSON, P.W.F. ET AL.: 'Metabolic Syndrome as a Precursor of Cardiovascular Disease and Type 2 Diabetes Mellitus' CIRCULATION vol. 112, 2005, pages 3066 - 3072
- GRUNDY, S.M. ET AL.: 'Diabetes and Cardiovascular Disease - A Statement for Healthcare Professionals from theAmerican Heart Association' CIRCULATION vol. 100, 1999, pages 1134 - 1146
- KRAUSS, R.M. ET AL.: 'Obesity - Impact on Cardiovascular Disease' CIRCULATION vol. 98, 1998, pages 1472 - 1476

## Description

### Technical Field of the Invention

The present invention relates generally to the use of a standardised aqueous extract of *Labisia pumila* leaves prepared by the method as disclosed in Malaysian patent application number PI 20054784, as a herbal medicine or as a botanical drug, and more particularly, to reduce the risk or progression of cardiovascular diseases by addressing factors that influence the pathophysiological changes (including regulation of various related genes) associated with insulin resistance, obesity, metabolic syndrome, diabetes and aging especially in women.

### Background of the Invention

With rapid urbanization and changing lifestyle, cardiovascular disease (CVD) is now the leading cause of death in many countries. Many factors are known to influence the degree to which an individual can develop CVD, these include age, gender, genetics and lifestyle, notably dietary habits.

Whilst healthy lifestyle habits such as exercise and a well-balanced diet are the best and most effective methods to prevent CVD, for women, estrogen has been known to play a significant role in protecting and maintaining not only the cardiovascular health but also the bone mass and mental cognition. These protective effects however, will be significantly reduced as the woman enters menopause or premature menopause a result of surgical removal of ovaries or following medical treatments such as chemotherapy and pelvic radiation therapy. On average, CVD kills five times more women than breast cancer.

Hence interventions, be it lifestyle, pharmacological or the use of complementary medicines such as herbal remedies could delay or even prevent CVD. There have been many studies that support the beneficial effects and use of dietary supplements like soy proteins, green tea and Chinese herbs like Gynostemma, Rhodiola and Ganoderma for maintaining cardiovascular health.

*Labisia pumila* or better known as "Kacip Fatimah" (also known as Selusoh Fatimah, Rumput Siti Fatimah, Akar Fatimah, Kachit Fatimah, Kachip Fatimah, Kachip Patimah, Kunchi Fatimah, Pokok pinggang, Rumput palis, Tadah mata hari, Mata pelandok rimba, Bunga belangkas hutan) is a Malay herb as disclosed in Burkhill (1993). Whilst cultivation is now possible, this herbaceous shrub can be found growing wildly on the forest floor only in Peninsular Malaysia and Kalimantan. For over 400 years, a decoction of the plant is traditionally taken to ease childbirth and during post-partum, to help to firm and tone vaginal and stomach muscles and to regain vitality. Additional uses include regulation of menstruation, relieve of menstrual pain, anti-flatulence, anti-dysentery and cures "sickness in the bones".

### Summary of the Invention

Accordingly, it is the primary object of the present invention to provide an oral use for standardised aqueous extract of *Labisia pumila* that can reduce the risk or progression of cardiovascular diseases and age-related health problems.

It is yet another object of the present invention to provide an oral use of the said standardized aqueous extract for influencing pathophysiological changes.

These and other objects of the present invention are accomplished by providing, a

standardised aqueous extract of *Labisia pumilal* for oral use in orally sufficient amount characterised in that said extract reduces the risk or progression of cardiovascular diseases and age-related health problems.
and
a standardised aqueous extract of *Labisia pumila* for use as defined, wherein the extract influences pathophysiological changes, including regulation of genes associated with insulin resistance, obesity, metabolic syndrome, diabetes and aging.

### Brief Description of the Figures

Figure 1 shows body weight changes in rats fed with standardised aqueous extract of *Labisia pumila.*
Figure 2 shows uterine integrity of rats fed with standardised aqueous extract of *Labisia pumila.*
Figure 3 shows glucose utilization of rats fed with standardised aqueous extract of *Labisia pumila.*
Figure 4 shows plasma leptin level in rats fed with standardised aqueous extract of *Labisia pumila.*
Figure 5 shows leptin expression in adipose tissue in rats fed with standardised aqueous extract of *Labisia pumila.*
Figure 6 shows leptin expression in liver tissue in rats fed with standardised aqueous extract of *Labisia pumila.*
Figure 7 shows adiponectin level in rats fed with standardised aqueous extract of *Labisia pumila.*
Figure 8 shows adiponectin level in adipose tissue in rats fed with standardised aqueous extract of *Labisia pumila.*
Figure 9 shows PPAR-gamma expression in adipose tissue in rats fed with standardised aqueous extract of *Labisia pumila.*
Figure 10 shows PPAR-gamma expression in liver tissue in rats fed with standardised aqueous extract of *Labisia pumila.*

### Detailed Description of the Invention

The present invention relates to the oral use of a standardised aqueous extract of *Labisia pumila* leaves prepared by the method as disclosed in Malaysian patent application number PI 20054784, as a herbal medicine or as a botanical drug, to reduce the risk or progression of cardiovascular diseases by addressing factors that influence the pathophysiological changes, including regulation of various related genes, associated with insulin resistance, obesity, metabolic syndrome, diabetes and aging especially in women.

The standardised aqueous extract of *Labisia pumila* leaves is not intended to replace pharmacological therapy but rather to emphasize that together with healthy lifestyle habits, the cumulative beneficial and preventive effects of this extract, especially when diligently taken, will be beneficial in maintaining health and therefore, lessen the risk of CVD and age-related health problems especially in women.

### Example 1

In one example, the effects of the extract on adipokines, glucose utilization, weight and uterine integrity in rats was studied.
Ovariectomized Sprague-Dawley rats were treated daily with standardised aqueous extract of *Labisia pumila* in the dose of 10 to 50 mg/kg/day for 30 days. The extract was dissolved in 2-3 ml distilled water and fed to the rats by oral gavage. At the end of the study, glucose level was determined using blood collected from the tail vein. The animals were later sacrificed and adipose fat, skeletal muscle and liver tissues excised and immediately processed for gene expression study. The plasma was stored frozen at -80°C until analysed. Uterine weight was recorded prior to being processed for histological examination. Enzyme- or radioimmunoassay kits were used to determine the levels of insulin and adipokines hormones in the plasma samples.

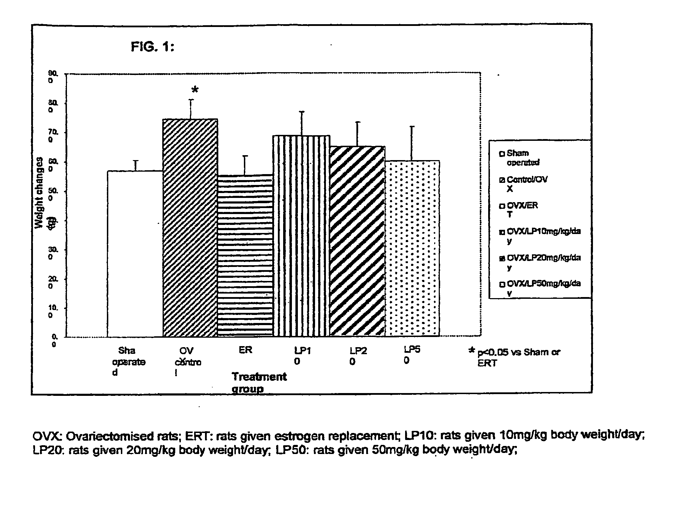

Estrogen-depleted rats showed significant increase in weight but in those given the extract, a dose-relationship was observed between the amounts of extract used and reduced weight gain (Fig. 1).

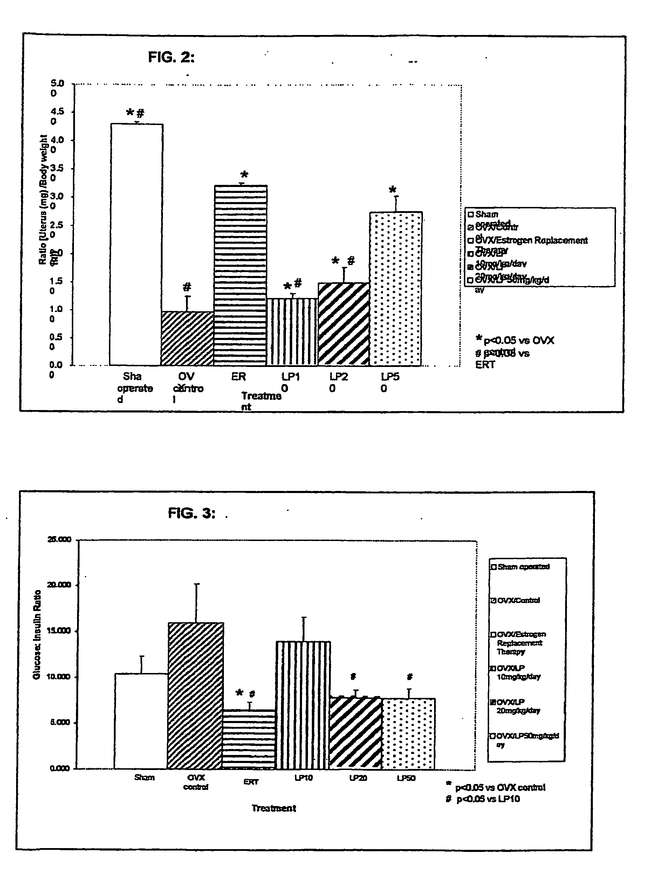

Uterine integrity of the treated animals was found to be restored with increasing dose of the extract (Fig. 2).

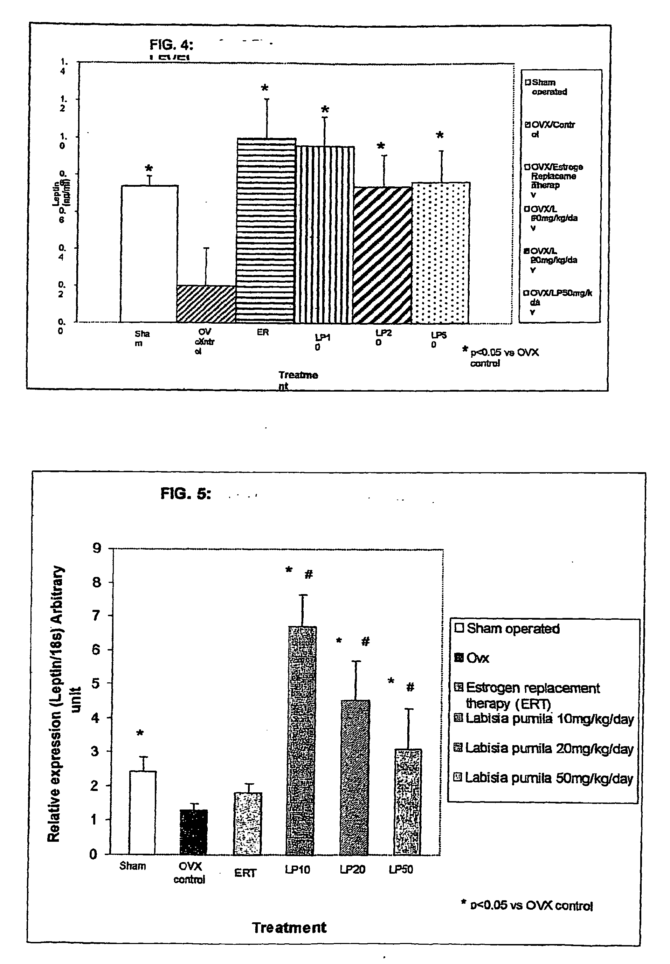

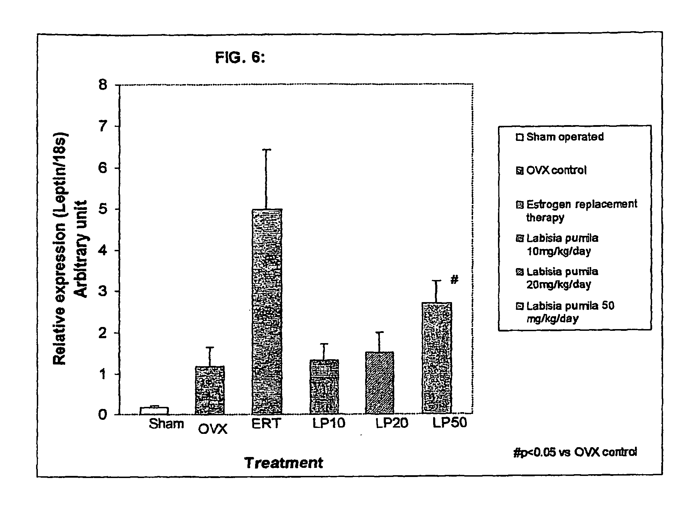

Compared to the control groups, animals given extract showed improved glucose utilization comparable to those on estrogen replacement (Fig. 3).
In rats given the extract, there was significant increase in plasma leptin level (Fig. 4) and leptin gene expression of the adipose (Fig. 5) and liver tissues (Fig. 6).

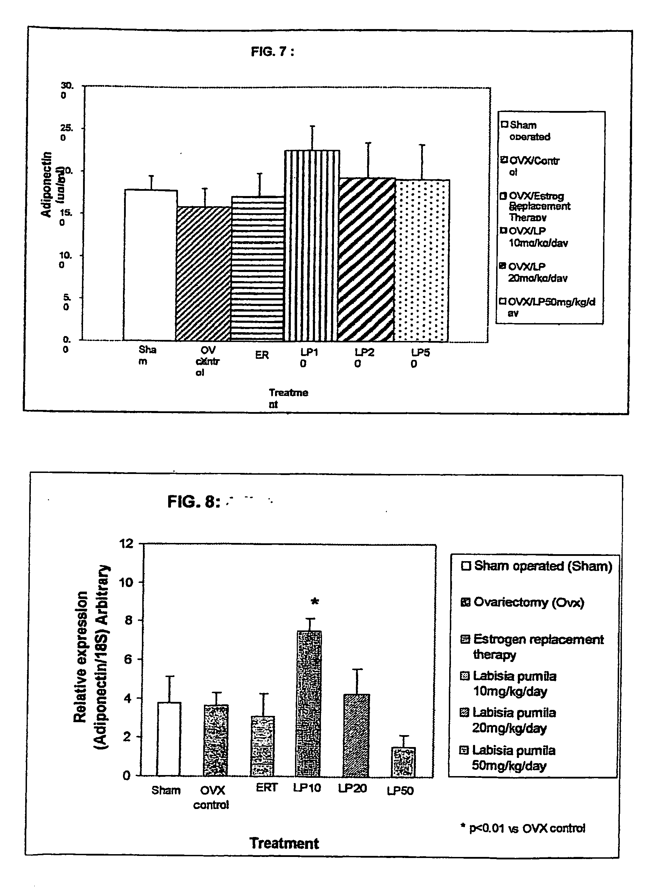

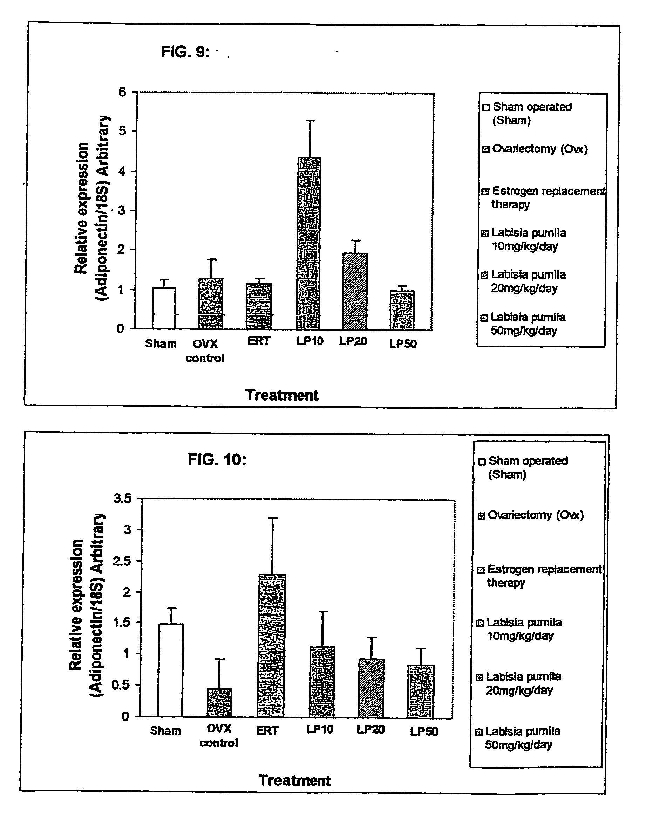

Similarly, rats fed with the extract had higher plasma adiponectin (Fig. 7) and increased adiponectin gene expression in the adipose tissues (Fig. 8).
The PPAR-gamma gene expression was also higher in the adipose (Fig. 9) and liver tissues of rats given the extract (Fig. 10).

### Example 2

In another example, a double-blind placebo controlled study was carried out using 116 healthy female volunteers aged 48-55 years old. The subjects received either 140 mg or 280 or 560 mg per day of the extract or placebo for 360 days. There was an overall improvement in lipids profile, statistically significant for triglycerides (p=0.003) and Apolipoprotein B (p=0.006) (Table 1).

**TABLE 1**

| Test | Placebo (n=32) | 140mg/day (n=29) | 280mg/day (n=32) | 560mg/day (n=25) | P value |
|---|---|---|---|---|---|
| Total cholesterol (mmol/L) | 5.4 | 5.5 | 5.0 | 5.0 | 0.077 |
| Triglycerides (mmol/L) | 2.02 | 1.24 | 1.30 | 1.16 | 0.003 |
| HDL-cholesterol (mmol/L) | 1.14 | 1.11 | 1.09 | 1.01 | 0.517 |
| LDL-cholesterol (mmol/L) | 3.55 | 3.73 | 3.29 | 3.41 | 0.347 |
| Apolipoprotein B (mg/dL) | 95.6 | 93.6 | 87.9 | 83.0 | 0.006 |

This result further support the claim that the aqueous extract of *Labisia pumila,* if taken diligently, can maintain the cardiovascular health and therefore, lessen the risk of CVD and age-related health problems especially in women.

### REFERENCES

Burkill, I.H 1993. A dictionary of the economic products of the Malay Peninsula. v. II (I-Z). Third print Kuala Lumpur: Ministry of Agriculture. 1311.
Lerner & KanneL Patterns in Coronary Heart Disease-Morbidity and Mortality in the Sexes: A 26-Year Follow-Up of the Framingham Population. Amer. Heart J 1986; 111:383-390.
Richeson, L. S. et al. Relative Contributions of Aging and Estrogen Deficiency to Postmenopausal Bone Loss. N. Eng. J. Med. 1984; 311: 1273-1275.
Sayegh, R et al. Impact of Hormone Replacement Therapy on the Body Mass and Fat Compositions of Menopausal Women: A Cross-Sectional Study, Menopause 1999; 6 (4): 312-315.
Kurzer M.S. Phytoestrogen supplement use by women. J Nutr. 2003; 133(6): 1983-1986.
Michael Blaha and Tom A Elasy. Clinical use of the metabolic syndrome: why the confusion? Clin Diab 2006; 24: 125-131
Reaven G. The metabolic syndrome or the insulin resistance syndrome? Different names, different concepts, and different goals. Endocrinol Metab Clinics North Am 2004; 33:283-303.
Abel Romero-Corral et al. Association of bodyweight with total mortality and with cardiovascular events in coronary artery diseases: a systematic review of cohort studies. Lancet 2006; 368: 666-678

It will of course be realised that whilst the above has been by way of an illustrative example of the invention, all such and other modifications and variations thereto, as would be apparent to persons skilled in the art, are deemed to fall within the broad scope and ambit of the invention as claimed herein.

## Claims

1. Standardized aqueous extract of *Labisia pumila* for oral use in orally sufficient amount for reducing the risk factors or progression of cardiovascular diseases,
and for reducing the risk factors or progression of age-related health problems selected from: insulin resistance, obesity, metabolic syndrome and diabetes.

2. Standardized aqueous extract for use according to claim 1, wherein the extract influences pathophysiological changes, including regulation of genes associated with insulin resistance, obesity, metabolic syndrome, diabetes and aging.

3. Standardized aqueous extract for use according to any one of claims 1 or 2, wherein the extract causes lowering of biochemical markers for lipids and apolipoproteins.

4. Standardized aqueous extract for use according to claim 1, wherein the extract regulates the level of adipokines and related gene expression in mammals.

5. Standardized aqueous extract for use according to claim 1, wherein the extract improves glucose utilization and regulates body weight in mammals.

6. Standardized aqueous extract for use according to of claim 1, wherein the extract maintains uterine wall integrity in mammals.

7. Standardized aqueous extract for use according to claims 1 or 2, wherein the extract reduces the risk factors of cardiovascular diseases and age-related health problems, selected from insulin resistance, obesity, metabolic syndrome and diabetes, in women.

8. Use of an orally sufficient amount of a standardized aqueous extract of *Labisia pumila* in the manufacture of a medicament for reducing the risk factors or progression of cardiovascular diseases, and for reducing the risk factors or progression of age-related health problems selected from insulin resistance, obesity, metabolic syndrome and diabetes.

9. Use of a standardized aqueous extract in the manufacture of a medicament, as according to claim 8, wherein the medicament is for use in regulating the level of adipokines and related gene expression in mammals.

10. Use of a standardized aqueous extract in the manufacture of a medicament, as according to claim 8, wherein the medicament is for use in improving glucose utilization and regulating body weight in mammals.

11. Use of a standardized aqueous extract in the manufacture of a medicament, as according to claim 8, wherein the medicament is for use in maintaining uterine wall integrity in mammals.

12. Use of a standardized aqueous extract in the manufacture of a medicament, as according to claim 8, wherein the medicament is for use in reducing the factors risk of cardiovascular diseases and age-related health problems, selected from insulin resistance, obesity, metabolic syndrome and diabetes, in women.

## Patentansprüche

1. Standardisierter wässriger Extrakt von *Labisia pumila* für orale Verwendung in oral ausreichender Menge zum Reduzieren der Risikofaktoren oder des Fortschritts von Herz-Kreislauf-Erkrankungen, und zum Reduzieren der Risikofaktoren oder des Fortschritts von altersbezogenen Gesundheitsproblemen, ausgewählt aus: Insulinresistenz, Fettleibigkeit, metabolisches Syndrom und Diabetes.

2. Standardisierter wässriger Extrakt zur Verwendung nach Anspruch 1, wobei der Extrakt pathophysiologische Veränderungen einschließlich von Regulation von Genen, die mit Insulinresistenz, Fettleibigkeit, dem metabolischen Syndrom, Diabetes und Altern assoziiert sind, beeinflusst.

3. Standardisierter wässriger Extrakt zur Verwendung nach einem der Ansprüche 1 oder 2, wobei der Extrakt Absenken von biochemischen Markern für Lipide und Apolipoproteine bewirkt.

4. Standardisierter wässriger Extrakt zur Verwendung nach Anspruch 1, wobei der Extrakt das Niveau von Adipokinen und von verwandter Geneexpression in Säugetieren reguliert.

5. Standardisierter wässriger Extrakt zur Verwendung nach Anspruch 1, wobei der Extrakt die Glukoseverwertung verbessert und das Körpergewicht in Säugetieren reguliert.

6. Standardisierter wässriger Extrakt zur Verwendung nach Anspruch 1, wobei der Extrakt die Uteruswandintegrität in Säugetieren aufrechterhält.

7. Standardisierter wässriger Extrakt zur Verwendung nach Anspruch 1 oder 2, wobei der Extrakt die Risikofaktoren von Herz-Kreislauf-Erkrankungen oder altersbezogenen Gesundheitsproblemen, ausgewählt aus Insulinresistenz, Fettleibigkeit, metabolisches Syndrom und Diabetes, in Frauen reduziert.

8. Verwendung einer oral ausreichenden Menge eines standardisierten wässrigen Extrakts von *Labisia pumila* bei der Herstellung eines Medikaments zum Reduzieren der Risikofaktoren oder des Fortschritts von Herz-Kreislauf-Erkrankungen und zum Reduzieren der Risikofaktoren oder des Fortschritts von altersbezogenen Gesundheitsproblemen, ausgewählt aus Insulinresistenz, Fettleibigkeit, metabolisches Syndrom und Diabetes.

9. Verwendung eines standardisierten wässrigen Extrakts bei der Herstellung eines Medikaments nach Anspruch 8, wobei das Medikament zur Verwendung bei der Regulation des Niveaus von Adipokinen und von verwandter Geneexpression in Säugetieren ist.

10. Verwendung eines standardisierten wässrigen Extrakts bei der Herstellung eines Medikaments nach Anspruch 8, wobei das Medikament zur Verwendung bei der Verbesserung der Glukoseverwertung und der Regulation des Körpergewichts in Säugetieren ist.

11. Verwendung eines standardisierten wässrigen Extrakts bei der Herstellung eines Medikaments nach Anspruch 8, wobei das Medikament zur Verwendung bei der Aufrechterhaltung der Uteruswandintegrität in Säugetieren ist.

12. Verwendung eines standardisierten wässrigen Extrakts bei der Herstellung eines Medikaments nach Anspruch 8, wobei das Medikament zur Verwendung bei der Reduzierung der Risikofaktoren von Herz-Kreislauf-Erkrankungen und altersbezogenen Gesundheitsproblemen, ausgewählt aus Insulinresistenz, Fettleibigkeit, metabolisches Syndrom und Diabetes, in Frauen ist.

## Revendications

1. Extrait aqueux normalisé de *Labisia pumila* pour utilisation en une quantité orale suffisante, destiné à réduire les facteurs de risque ou la progression de maladies cardiovasculaires et destiné à réduire les facteurs de risque ou la progression de problèmes de santé associés à l'âge sélectionnés parmi: l'insulino-résistance, l'obésité, le syndrome métabolique et le diabète.

2. Extrait aqueux normalisé à utiliser selon la revendication 1, où l'extrait influence des changements pathophysiologiques y compris la régulation de gènes associés à l'insulino-résistance, à l'obésité, au syndrome métabolique, au diabète et au vieillissement.

3. Extrait aqueux normalisé à utiliser selon l'une quelconque des revendications 1 ou 2, où l'extrait cause un abaissement des marqueurs biochimiques pour les lipides et les apolipoprotéines.

4. Extrait aqueux normalisé à utiliser selon la revendication 1, où l'extrait régule le taux d'adipokines et de l'expression génique associée chez les mammifères.

5. Extrait aqueux normalisé à utiliser selon la revendication 1, où l'extrait améliore l'utilisation du glucose et régule le poids corporel chez les mammifères.

6. Extrait aqueux normalisé à utiliser selon la revendication 1, où l'extrait maintient l'intégrité de la paroi utérine chez les mammifères.

7. Extrait aqueux normalisé à utiliser selon les revendications 1 ou 2, où l'extrait réduit les facteurs de risque de maladies cardiovasculaires et les problèmes de santé associés à l'âge, sélectionnés parmi l'insulino-résistance, l'obésité, le syndrome métabolique et le diabète chez la femme.

8. Utilisation d'une quantité orale suffisante d'un extrait aqueux normalisé de *Labisia pumila* dans la fabrication d'un médicament pour réduire les facteurs de risque ou la progression de maladies cardiovasculaires et pour réduire les facteurs de risque ou la progression de problèmes de santé associés à l'âge sélectionnés parmi l'insulino-résistance, l'obésité, le syndrome métabolique et le diabète.

9. Utilisation d'un extrait aqueux normalisé dans la fabrication d'un médicament, selon la revendication 8, où le médicament est destiné à être utilisé pour réguler le taux d'adipokines et de l'expression génique associée chez les mammifères.

10. Utilisation d'un extrait aqueux normalisé dans la fabrication d'un médicament, selon la revendication 8, où le médicament est destiné à être utilisé pour améliorer l'utilisation du glucose et réguler le poids corporel chez les mammifères.

11. Utilisation d'un extrait aqueux normalisé dans la fabrication d'un médicament, selon la revendication 8, où le médicament est destiné à être utilisé pour maintenir l'intégrité de la paroi utérine chez les mammifères.

12. Utilisation d'un extrait aqueux normalisé dans la fabrication d'un médicament, selon la revendication 8, où le médicament est destiné à être utilisé pour réduire les facteurs de risque de maladies cardiovasculaires et les problèmes de santé associés à l'âge, sélectionnés parmi l'insulino-résistance, l'obésité, le syndrome métabolique et le diabète, chez la femme.
